(19) Europäisches Patentamt

European Patent Office

Office européen des brevets

(11) **EP 3 421 971 A1**

(12) **EUROPEAN PATENT APPLICATION**

(43) Date of publication:
**02.01.2019   Bulletin 2019/01**

(51) Int Cl.:
**G01N 21/25** (2006.01)      **G01J 3/46** (2006.01)
**G01J 3/50** (2006.01)      **G01N 33/02** (2006.01)

(21) Application number: **17178041.4**

(22) Date of filing: **27.06.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Koninklijke Philips N.V.
5656 AE Eindhoven (NL)**

(72) Inventors:
  • **JIN, Yafang
    5656 AE Eindhoven (NL)**
  • **LUO, ZhongChi
    5656 AE Eindhoven (NL)**
  • **LU, Weihua
    5656 AE Eindhoven (NL)**
  • **FENG, Haitao
    5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **DEVICE AND METHOD FOR DETERMINING SUGAR CONTENT INFORMATION FOR A FOOD ITEM**

(57)   The present application relates to a device for determining sugar content information for a food item during or after cooking. It is described to receive the food item and receive a food type of the food item. The color change of the food item is measured during or after cooking. Sugar content information for the food item is determined on the basis of the food type and the measured color change. The present application also relates to a heating appliance, a method for determining sugar content information and a computer program element.

FIG. 1

EP 3 421 971 A1

## Description

FIELD OF THE INVENTION

The present invention relates to the field of sugar content determination, and more particularly to a device for determining sugar content information for a food item, to a heating appliance for cooking a food item, to a method for determining sugar content information for a food item, as well as to a computer program element.

BACKGROUND OF THE INVENTION

Sugar is the generic name for sweet, soluble carbohydrates, many of which are used in food. There are various types of sugar derived from different sources. Simple sugars are called monosaccharides and include glucose (also known as dextrose), fructose, and galactose. The "table sugar" or "granulated sugar" most customarily used as food is sucrose, a disaccharide of glucose and fructose. Sugar is used in prepared foods (e.g., cookies and cakes) and it is added to some foods and beverages (e.g., coffee and tea). In the body, sucrose is hydrolyzed into the simple sugars fructose and glucose. Other disaccharides include maltose from malted grain, and lactose from milk. Longer chains of sugars are called oligosaccharides or polysaccharides.

Sugar consumption is extremely high in our daily life. According to data from the U.S. in 2008, people are consuming over 60 pounds (28 kg) of added sugar per year and this does not include fruit juices. In 2008 the average intake was 76.7 grams per day, which equals 19 teaspoons or 306 calories. According to this study, sugar consumption went down by 23% between the years 2000 and 2008, mainly because people drank less sugar-sweetened beverages. However, current intake levels are still way too high and are a key player in making people fat and sick. Specifically, excess sugar consumption has been associated with obesity, type II diabetes, cardiovascular disease, certain cancers, tooth decay, non-alcoholic fatty liver disease and a lot more. To support a satisfactory nutritional status of the population, the knowledge of sugar content in food is important for people, especially for those who are on a diet.

There are several nutrient databases available to provide the sugar content of food. However, for one food type, the databases only contain the average sugar content value. The specific composition of each foodstuff varies due to growing, harvesting, storing and processing conditions. Besides, chemical analysis of sugar content in food cannot be applied in practical home-cooking situations since it only can be carried out by professional operator in lab and need relative long time.

SUMMARY OF THE INVENTION

It would be advantageous to have improved means to automatically and conveniently determine the sugar content information for a food item.

The object of the present invention is solved with the subject matter of the independent claims; wherein further embodiments are incorporated in the dependent claims. It should be noted that the following described aspects and examples of the invention apply also for the device for determining sugar content information for a food item, heating appliance for cooking a food item, method for determining sugar content information for a food item, and computer program element.

According to a first aspect, there is provided a device for determining sugar content information for a food item during or after cooking. The device may include an object receiving unit for receiving the food item, an input unit for receiving a food type of the food item, a color measuring unit for measuring color change of the food item during or after cooking, and a processing unit configured to determine sugar content information for the food item on the basis of the food type and the measured color change.

During cooking and/or heating, browning color appears, as the result of the Maillard reaction and caramelization. Maillard reaction is non-enzymatic browning due to the reaction between the reducing sugars and the $\alpha$-amino groups of amino acids. Reducing sugars are essential ingredients in these actions, providing the carbonyl groups for interaction with the free amino groups of amino acids, peptides, and proteins. Caramelization is another example of non-enzymatic browning involving the degradation of sugars and generally proceeds simultaneously with the Maillard reaction. Caramelization of sugars contributes markedly to the production of brown pigments. So during high temperature cooking (e.g., >130°C), for a given food type, the sugar content is strongly linked to non-enzymatic browning under specific cooking conditions (pH, temperature, humidity, etc.). Put it in another way, with higher sugar content, the browning is much deeper. In view of this, it would be feasible to predict sugar content (%) by making color and/or image analysis of the browning level of the food item.

With the device in the context of the present application, the user may obtain the sugar content information easily and conveniently with no chemical analysis or any other efforts. Further, based on the experiments made by the inventors, the correlation of sugar content and color change is relatively high with variance larger than 0.95. Thus, the device for determining sugar content information could achieve a high accuracy.

The sugar content information that is determined can be used to track people's daily intake so as to help people keep a well-balanced diet. This is especially helpful if no nutrient compositions of the food item are available.

In one embodiment of the present application, the device can further include a weight sensor configured to measure weight of the food item in the object receiving unit as a function of time.

The measurement of weight of the food item as a function of time would be helpful to determine the food dimension information for that food item. For example, for various cultivars of potatoes, experiments can be performed. Correlation information for these different food types can then be experimentally determined, enabling a database of correlation information to be generated. Then, from the measured weight of the food item as a function of time, with knowledge of the food type for that food item, in effect a specific piece of "correlation data" is generated for that food item. This specific piece of correlation data (the measured weight of the food item as a function of time), for that food type, can then be referenced to the subset of the database of correlation information that relates to the same food type. In an example, a match of this correlation data with the correlation information in the database selects the food dimension information in the database, which resulted in the matching information. This therefore determines the food dimension information for the food item. In this way, the device enables food dimension information for a particular food type to be determined from the measured weight as a function of time during, for example, air frying.

The food dimension information that is determined can then be used to determine suitable cooking parameters, such as a cooking temperature or temperature profile, and/or a cooking duration. In other words, after the food dimension information has been determined, a more suitable cooking process can be selected. The determined food dimension information can also be used to track the level of doneness of the food item.

In this manner, on the basis of information relating to the food type and on a measurement of the weight of a food item, dimension information can be calculated. The food dimension information can then be used to determine how best the food item can be cooked and/or determine when the food item has been correctly cooked (correctly cooked here equates to "doneness"). In other words, food dimension information is automatically determined, and this is useable to allow a later heating strategy to be adjusted accurately and in an uninterrupted fashion.

Furthermore, dimension information can be determined whatever the shape the food item takes. In this way, food dimension information enables cooking safety and efficiency of cooking to be improved, and enables food items to be better cooked to the required level of "doneness".

Furthermore, it is to be noted that the potential non-evaporative weight change for example due to dripping and spillage of water, melting of fat or flow of oil will lead to errors. The measured weight as a function of time is desired to be due primarily to water evaporation. In another word, there can be dripping or spillage of water, and melting of fat and flow of oil, but it is desired that these lead to no weight change. By measuring the weight of the object receiving unit, measurement of weight change due to water evaporation is facilitated. This is because, potential non-evaporative weight change will not affect the weight measurement because the water and fat can be contained within the object receiving unit and where such dripping, spillage of water and melted fat will not lead to change in weight. The change in weight is then determined primarily for the evaporation of water, thereby enabling a more accurate determination of food dimension information.

In one embodiment of the present application, the processing unit is further configured to determine an endpoint of cooking at least based on the weight loss rate of the food item (dependent on weight change of the food item as a function of time), which enables cooking safety and efficiency of cooking to be improved, and enables food items to be better cooked to the required level of "doneness".

In one embodiment of the present application, the color measuring unit is an image recognition system or a color sensor fixed to the object receiving unit. It would be appreciated by persons skilled in the art that the color measuring unit can be any kinds of measuring unit which is capable of measuring color change of the food item. Furthermore, the color measuring unit can be separate from the object receiving unit in an alternative solution.

In one embodiment of the present application, the color measuring unit is configured to take pictures of the food item, obtain RGB data by analyzing the taken pictures and convert the RGB data to CIE-L*a*b* coordinates.

There are no simple formulas for conversion between RGB values and L*a*b*, because the RGB color models are device-dependent. The RGB values first have to be transformed to a specific absolute color space, such as sRGB or Adobe RGB. This adjustment will be device-dependent, but the resulting data from the transform will be device-independent, allowing data to be transformed to the CIE 1931 color space and then transformed into L*a*b*.

In this manner, the parameters related to color change or browning level, such as red-green variation (a*) or hue (h*) can be determined. With the knowledge of food type and color change, the processing unit can then be configured to determine the sugar content information of the food item.

Alternatively, the color measuring unit is configured to take pictures of the food item and obtain RGB or CMYK values by analyzing the taken pictures.

In this manner, the parameters related to color change or browning level, such as R values can be determined. With the knowledge of food type and color change, the processing unit can then be configured to determine the sugar content information of the food item.

In one embodiment of the present application, the device can further include a recipe guide unit for displaying a recipe menu including a list of the food types to a user for selection. This allows the user to choose food type from a list of entries which is convenient. In this manner, the input unit can be configured to receive the food type from the recipe guide unit.

In one embodiment of the present application, the processing unit is further configured to store correlation information between the sugar content information and the measured color change for a particular food type. The correlation information can be empirical data obtained, for example from experiments. As an example, a number of samples (or examples) of a particular food type and having known sugar content information can be heated while the color change being measured. The color change for these different samples having different sugar content information can then be determined, and a graph (or tabled information) (for example) of the sugar content information as a function of color change plotted (or tabulated). Then, by cross correlating with the graphical (or tabled) information the sugar content information for the food item can be determined. Here, plotting or tabulation of data is simply used to indicate that the data can be used to specify a correlation, where that correlation can be implemented within the processing unit without the need for a physical graph for example, as would be appreciated by the skilled person.

In one embodiment of the present application, the device may further include a shaking unit for shaking the food item. This is particularly useful to make the color change of the food item more even. For example, the shaking unit works when weight loss reaches 25% and 40% respectively during the cooking.

In one embodiment of the present application, the device is configured to heat the food item or subject the food item to air convection; or the device or its object receiving unit is configured to heat the food item by means of convectional aided heating. In this way, the color change of the food item shall be more even than those subject to other cooking methods. This could provide a more accurate result for sugar content determination. Additionally, the free water on the surface of the food item could be evaporated more easily. Thus, for a food item of given amount (e.g. 30g), a clear weight change (e.g. 2-3g) can be observed earlier. This could reduce the requirements for the sensor used. Meanwhile, this could shorten the time and reduce the temperature needed for the determination of the sugar content information.

According to a second aspect, there is provided a heating appliance for cooking a food item, comprising the device for determining sugar content information according to the above described first aspect and optionally one or more of any of the described embodiments.

In one embodiment of the present application, the heating appliance is an air fryer or an oven.

In this manner, when the air fryer or oven forms part of a smart cooking apparatus, a separate sensor such as a Near Infra-Red (i.e., NIR) sensor for monitoring the sugar content is not required.

Additionally, the air fryer or oven can control the air speed, and/or control the air temperature, and/or control the air humidity entering the air fryer or oven, thereby enabling a more accurate determination of sugar content information of a food item.

According to a third aspect, there is provided a method for determining sugar content information for a food item during or after cooking. The method includes receiving the food item; receiving a food type for the food item; measuring color change of the food item during or after cooking; and determining sugar content information for the food item on the basis of the food type and the measured color change.

In one embodiment of the present application, the method further includes measuring weight of the food item as a function of time.

In one embodiment of the present application, the method further includes determining an endpoint of cooking at least based on the weight loss rate of the food item dependent on weight change of the food item as a function of time.

According to another aspect, there is provided a computer program element controlling a device or heating appliance as previously described which, in the computer program element is executed by processing unit, is adapted to perform the method steps as previously described.

According to another aspect, there is provided a computer readable medium having stored computer element as previously described.

Advantageously, the benefits provided by any of the above aspects equally apply to all of the other aspects and vice versa.

The above aspects and examples will become apparent from and be elucidated with reference to the embodiments described hereinafter.

BRIEF DESCRIPTION OF THE DRAWINGS

Exemplary embodiments will be described in the following with reference to the following drawings:

Figs. 1 to 4 show a schematic set up of a device for determining sugar content information for a food item during or after cooking according to various embodiments of the present application;
Fig. 5 shows a method for determining sugar content information for a food item during or after cooking according to one embodiment of the present application; and
Figs. 6a and 6b show fitting curves for different sugar content information according to embodiments of the present application.

DETAILED DESCRIPTION OF THE EMBODIMENTS

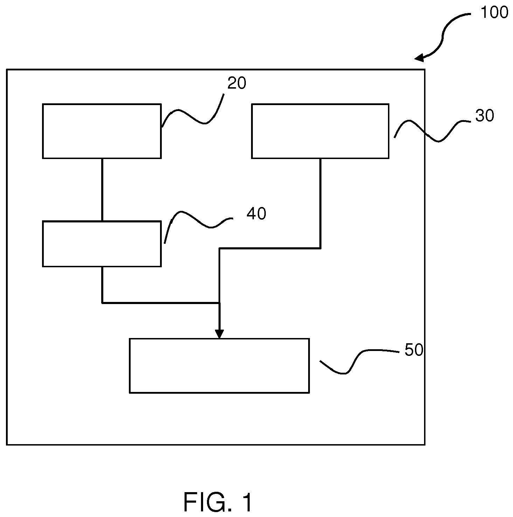

Fig. 1 shows an example of a device 100 for determining sugar content information for a food item during or after cooking according to one embodiment of the present application. The device 100 includes an object receiving unit 20, an input unit 30, a color measuring unit 40 and a processing unit 50. The object receiving unit 20 is configured to receive a food item. The input unit 30 is configured to receive a food type for the food item. The color measuring unit 40 is configured to measure color change of the food item during or after cooking. The processing unit 50 is configured to then determine sugar content information for the food item on the basis of the food type and the measured color change.

In this way, the device 100 enables sugar content information for a particular food type to be determined from measured color change during or after cooking. With this device 100, the user may obtain the sugar content information easily and conveniently with no chemical analysis or any other efforts. The sugar content information that is determined can be used to track people's daily intake so as to help people keep a well-balanced diet. This is especially helpful if no nutrient compositions of the food item are available.

In an example, the object receiving unit 20 is configured to be heated. In this manner, the food item can be heated and browning color appears as the result of the Maillard reaction and caramelization. In an example, the object receiving unit 20 is configured to be held at a stabilized temperature for a period of time over which the color change of the food item is measured. In an example, the object receiving unit 20 is configured to be heated thereby increasing the temperature inside the object receiving unit 20 and then held at a stabilized temperature, and wherein the color change of the food item is measured over the period when the object receiving unit 20 suffered an increase in temperature and was held at a stabilized temperature. In other words, the food item can be placed into an object receiving unit 20 that is "preheated" to a temperature at which the color change is measured, or the food item can be placed into an object receiving unit 20 that is then heated and is then held at a stabilized temperature, with the color change being measured. In an example, the object receiving unit 20 is not held at a stabilized temperature, but is just heated and the color change measured.

In an example, a user interacts with the input unit 30 to indicate the food type, such as entering that the food type is chips, fish, or meat, or more specifically pork or steak or chicken or lamb or salmon or cod, or a specific vegetable type, or a particular type of product such as meat balls, or burgers, or sausages, or more specifically, beef meat balls, turkey meat balls, beefburgers, pork sausages, or a particular cut such as chicken thigh, chicken breast "whole chicken", Ribeye steak, rump steak - for example. In other words, in an example the input unit 30 is configured to receive a food type through a user entering the food type into the input unit.

The term "food type" here means any type of food for which sugar content information can be determined as a function of color change. Here food type can mean common food species such as broccoli, chicken, carrot, fish (and can mean specific types of fish e.g. salmon, cod), or potato or food such as French fries, or chips. The food type can also refer to a food item from different cultivars or with different pre-treatments. Therefore, the skilled person will appreciate that food type can include the specie of food, for example potato, and can further include the specific cultivar, e.g., Shepody.

In an example, the input unit 30 receives the food type automatically, for example through a camera acquiring an image of the food item and image processing being used to determine the food type for that food item, with this information being passed to the input unit 30.

In an example, the color measuring unit 40 is configured to measure color change of the food item when the food item is put into the object receiving unit 20 during cooking with a stabilized temperature for a period of time. In an example, the color measuring unit 40 is configured to measure color change of the food item after cooking, e.g., when the temperature of the object receiving unit 20 for receiving the food item starts to fall.

The term "color change" here means any change of color which could be used to predict sugar content information. For example, color change can refer to red-green variation (a*) or hue (h*) in the CIE-L*a*b* system.

In an example, the color measuring unit 40 is configured to take pictures of the food item, obtain RGB data by analyzing the taken pictures and convert the RGB data to CIE-L*a*b* coordinates. There are no simple formulas for conversion between RGB values and L*a*b*, because the RGB color models are device-dependent. The RGB values first have to be transformed to a specific absolute color space, such as sRGB or Adobe RGB. This adjustment will be device-dependent, but the resulting data from the transform will be device-independent, allowing data to be transformed to the CIE 1931 color space and then transformed into L*a*b*. In this manner, the parameters related to color change or browning level, such as red-green variation (a*) or hue (h*) can be determined.

Alternatively, the color measuring unit 40 is configured to take pictures of the food item and obtain RGB or CMYK values by analyzing the taken pictures. In this manner, the parameters related to color change or browning level, such as R values can be determined.

On the basis of the information relating to the food type and on a measurement of the color change of a food item, sugar content information can be determined by the processing unit 50.

In an example, the processing unit 50 is further configured to store correlation information between the sugar content information and the measured color change for a particular food type. The correlation information can be empirical data obtained, for example from experiments. For example, a number of samples (or examples) of a particular food type and having known sugar content information can be heated while the color change being measured. For another example, the sugar content information for a particular food type can be obtained by chemical analysis. The color change for these different samples having different sugar content information can then be determined, and a graph (or tabled information) (for example) of the sugar content information as a function of color change plotted (or tabulated). Then, by cross correlating with the graphical (or tabled) information the sugar content information for the food item can be determined. Here, plotting or tabulation of data is simply used to indicate that the data can be used to specify a correlation, where that correlation can be implemented within the processing unit 50 without the need for a physical graph for example, as would be appreciated by the skilled person.

In an example, the temperature within the object receiving unit 20 is substantially constant during measurement of the color change of the food item.

It is seen from Fig. 1 that the color measuring unit 40 is separate from the object receiving unit 20. However, persons skilled in the art would appreciate that the color measuring unit 40 can be an integral part of the object receiving unit 20.

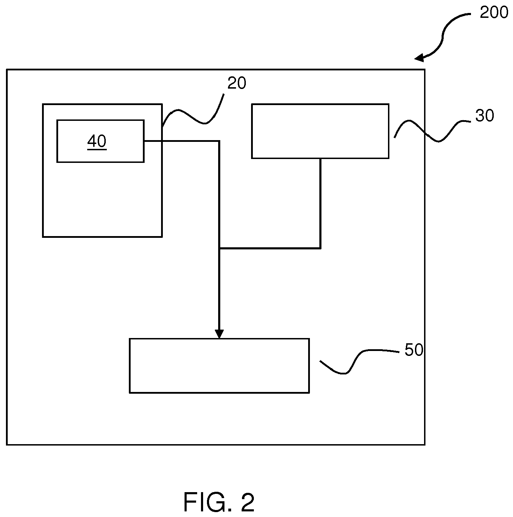

As shown in Fig.2, a device 200 for determining sugar content information for a food item during or after cooking according to another embodiment of the present application is depicted. Similar to Fig. 1, the device 200 includes an object receiving unit 20, an input unit 30, a color measuring unit 40 and a processing unit 50. The object receiving unit 20 is configured to receive a food item. The input unit 30 is configured to receive a food type for the food item. The color measuring unit 40 is configured to measuring color change of the food item during or after cooking. The processing unit 50 is configured to then determine sugar content information for the food item on the basis of the food type and the measured color change. But contrary to Fig.1, the color measuring unit 40 is integrated into the object receiving unit 20. In an example, the color measuring unit 40 is an image recognition system or a color sensor fixed to the object receiving unit 20. It would be appreciated by persons skilled in the art that the color measuring unit 20 can be any kinds of measuring unit which is capable of measuring color change of the food item.

In this way, the device 200 enables sugar content information for a particular food type to be determined from measured color change during or after cooking. With this device 200, the user may obtain the sugar content information easily and conveniently with no chemical analysis or any other efforts. The sugar content information that is determined can be used to track people's daily intake so as to help people keep a well-balanced diet. This is especially helpful if no nutrient compositions of the food item are available.

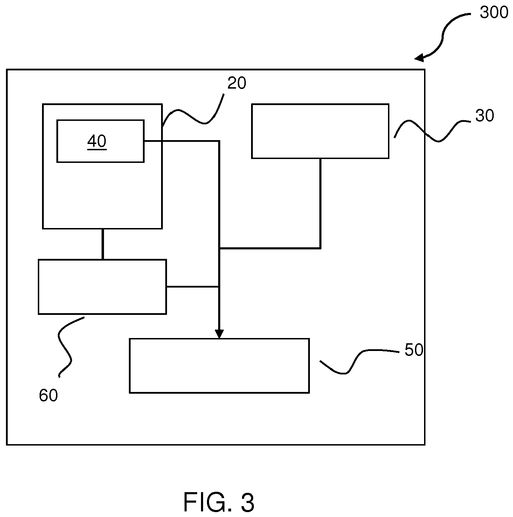

In one embodiment of the present application, as shown in Fig.3, a device 300 for determining sugar content information for a food item during or after cooking is shown. Compared with the device 200 as shown in Fig. 2, the device 300 further includes a weight sensor 60 configured to measure weight of the food item in the object receiving unit 20 as a function of time.

In an example, the weighing sensor 60 is used to calculate the amount of sugar (g) based on the determined sugar content (%) of a particular food item and the weight of the food item measured. In an example, the weighing sensor 60 is also used to indicate the change to the weight, and through the sensed change in weight along with the food type input by the user, the dimension factor (or the ratio of surface to volume) can be calculated. The dimension factor that is determined can then be used to determine suitable cooking parameters, such as a cooking temperature or temperature profile, and/or a cooking duration.

In an example, the measured weight of the food item is converted into a relative weight, for example leading to a percentage weight loss as a function of time. In other words, a starting weight is measured and subsequent weights are divided by the starting weight to determine a relative weight. In this manner, the processing unit 50 can be configured to determine an endpoint of cooking at least based on the percentage weight loss of the food item (dependent on weight change of the food item as a function of time), which enables cooking safety and efficiency of cooking to be improved, and enables food items to be better cooked to the required level of "doneness".

It is to be noted that the potential non-evaporative weight change for example due to dripping and spillage of water, melting of fat or flow of oil will lead to errors. The measured weight as a function of time is desired to be due primarily to water evaporation. In another word, there can be dripping or spillage of water, and melting of fat and flow of oil, but it is desired that these lead to no weight change. By measuring the weight of the object receiving unit, measurement of weight change due to water evaporation is facilitated. This is because, potential non-evaporative weight change will not affect the weight measurement because the water and fat can be contained within the object receiving unit and where such dripping, spillage of water and melted fat will not lead to change in weight.

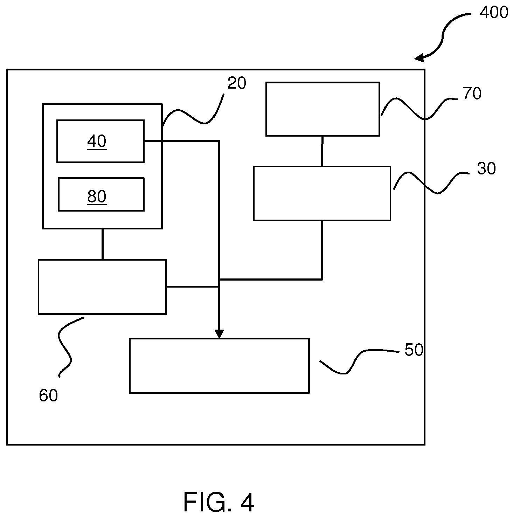

Turning to Fig. 4, a schematic set up of a device 400 for determining sugar content information for a food item during or after cooking is shown. Compared with the device 300 as shown in Fig. 3, the device 400 further includes a recipe guide unit 7 and a shaking unit 80. The recipe guide unit 70 is adapted for being communicately coupled with the input unit 30 and is configured for displaying a recipe menu including a list of the food types to a user for selection. In this manner, the input unit 30 can receive the food type from the recipe guide unit 70 which allows the user to choose food type from a list of entries.

In Fig. 4, the shaking unit 80 is shown within the object receiving unit 20 and is used for shaking the food item, which is particularly useful to make the color change of the food item more even. Yet, persons skilled in the art can appreciate that the shaking unit 80 can be placed at any appropriate place to shake the food item, not limited to be within the object receiving unit 20.

In various embodiments of the present application, the device for determining sugar content information can operate with air/oil convectional (both natural and forced) aided heating, since color change will be relatively fast and apparent for these cooking methods. Particularly, the color change of the food item shall be more even in convectional aided heating than that subject to other cooking methods, contributing to a more accurate result for sugar content determination.

According to one embodiment of the present application, the above-mentioned device(s) 100, 200, 300 and 400 can be considered to be included in a heating appliance for cooking a food item. In an example, the heating appliance is an air fryer or an oven.

In this manner, when the air fryer or oven forms part of a smart cooking apparatus, a separate sensor such as a Near Infra-Red (i.e., NIR) sensor for monitoring the sugar content is not required.

Additionally, the air fryer or oven can control the air speed, and/or control the air temperature, and/or control the air humidity entering the air fryer or oven, thereby enabling a more accurate determination of sugar content information of a food item.

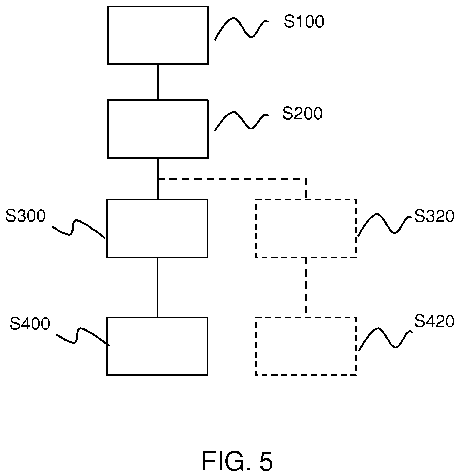

Fig. 5 shows a method for determining sugar content information for a food item during or after cooking according to one embodiment of the present application. The method includes:

In a receiving step S100, a food item is received.
In a receiving step S200, a food type for the food item is received.
In a measuring step S300, a color change of the food item during or after cooking is measured.
In a determining step S400, sugar content information is determined for the food item on the basis of the food type and the measured color change.

Additionally, the method may include a measuring step S320 in which a weight of the food item as a function of time is measured. Furthermore, the method may include a determining step S420 in which an endpoint of cooking is determined at least based on the weight loss rate of the food item.

An example of obtaining correlation information between sugar content information and color change for a food type will now be described in greater details.

For homemade potato fries, sugar content of potato tubers varies between cultivars, during storage time and pre-treatment. Glucose, and fructose are the major monosaccharide sugars in potato tubers with a concentration of 0.15-1.5%. Sucrose (0.4-6.6%) is a non-reducing disaccharide. To establish a dataset of empirical correlation information between sugar content information and color change for various food types, experiments were made for potatoes from different cultivars (potatoes from City shop, Shepody). Potatoes were peeled and cut into cuboid fries (about 1cm*1cm*7cm). Different pre-treatments were then carried out as followed: 1: soaking in room temperature water for 30 min, 2: bleaching in 60 °C water bath for 30 min, 3: treated with ultrasonic for 30 min, 4: low temperature (60 °C) steam for 30 min. Before heating, the fries (about 400g) were pat dry with paper towel, and mixed with about 4.68g oil. Then the fries were put into the air fryer XL (180 °C), and all cooked to the same doneness level (60% weight loss). During cooking, when weight loss reached 25% and 40%, the fries were shaked to make the color and texture more even.

The weighting sensor is put under the object receiving unit or the whole device to measure the water loss during cooking caused by evaporation, and to determine the endpoint of cooking. During or after cooking, pictures of the homemade fries were taken under the same lighting condition. And the sugar content of the fries with different cultivars or with different pre-treatments were tested by chemical methods.

There are differences in the browning level of these two kind of homemade fries. The sugar content of potatoes from City shop after pre-treated with 60 °C water bath is 0.37%, and the sugar content of Shepody potatoes after pre-treated with room temperature water soaking is 0.02%. It can be expected that with higher sugar content, the browning is much deeper.

For color analysis, these taken pictures are analyzed by the Image software to get the RGB data. And for each pictures, the average of three measurements in different areas is used. Then the RGB data is converted to CIE-L*a*b* coordinates. The CIE-L*a*b* system is composed of: luminosity (L*), red-green variation (a*), yellow-blue variation (b*).

Moreover, hue (h*) can be obtained by the following equations:

$$h^* = \text{arctg}\,(a^*/b^*)$$

It can be appreciated that the values of coordinate a*, and values of h*, are related to browning.

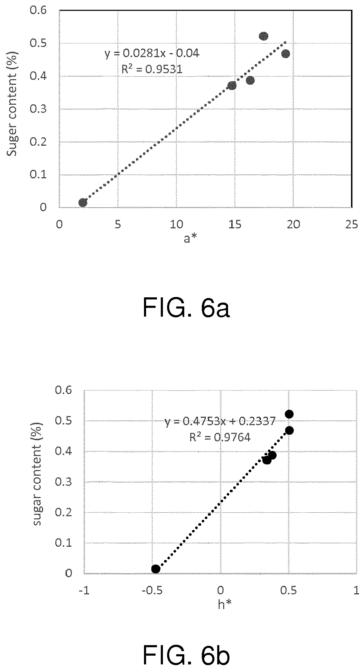

By making a statistic survey, as indicated in Figs. 6a and 6b, the sugar content of homemade potato fries with different cultivars or with different pre-treatments are obtained. It is seen that the sugar content is correlated with the value of coordinate a*, and the corresponding value of h*. The correlation of sugar content and color change such as red-green variation a* and hue h* is relatively high with variance larger than 0.95. Thus, a high accuracy for predicting sugar content (%) can be achieved.

In the manner described above, any integrated unit of a heating appliance (cooker for example) or a stand-alone sensing unit can use the color change obtained during or after cooking to derive sugar content information for the item being cooked. The user can thus obtain the sugar content information easily and conveniently with no chemical analysis or any other efforts. The sugar content information that is determined can be used to track people's daily intake so as to help people keep a well-balanced diet. This is especially helpful if no nutrient compositions of the food item are available.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described device and/or oven. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items recited in the claims. The mere fact that certain measures are re-cited in mutually different dependent claims does not indicate that a combination of these measures cannot be used to advantage.

Any reference signs in the claims should not be construed as limiting the scope.

## Claims

1. A device (100, 200, 300, 400) for determining sugar content information for a food item during or after cooking, comprising:

   an object receiving unit (20) for receiving the food item;
   an input unit (30) for receiving a food type of the food item;
   a color measuring unit (40) for measuring color change of the food item during or after cooking; and
   a processing unit (50), configured to determine sugar content information for the food item on the basis of the food type and the measured color change.

2. The device (100, 200, 300, 400) according to claim 1, further comprising a weight sensor (60) configured to measure weight of the food item in the object receiving unit as a function of time.

3. The device (100, 200, 300, 400) according to claim 2, wherein the processing unit (50) is further configured to determine an endpoint of cooking at least based on the weight loss rate of the food item which is dependent on weight change of the food item as a function of time.

**4.** The device (100, 200, 300, 400) according to any preceding claim, wherein the color measuring unit (40) is an image recognition system or a color sensor fixed to the object receiving unit (20).

**5.** The device (100, 200, 300, 400) according to any preceding claim, wherein the color measuring unit (40) is configured to take pictures of the food item, obtain RGB data by analyzing the taken pictures and convert the RGB data to CIE-L*a*b* coordinates.

**6.** The device (100, 200, 300, 400) according to any preceding claim, further comprising a recipe guide unit (70) for displaying a recipe menu including a list of the food types to a user for selection, wherein the input unit (30) is configured to receive the food type from the recipe guide unit (70).

**7.** The device (100, 200, 300, 400) according to any preceding claim, wherein the processing unit (50) is further configured to store correlation information between the sugar content information and the measured color change for a particular food type.

**8.** The device (100, 200, 300, 400) according to any preceding claim, further comprising a shaking unit (80) for shaking the food item to make the color change even.

**9.** The device (100, 200, 300, 400) according to any preceding claim, wherein the device (100, 200, 300, 400) is configured to heat the food item or subject the food item to air convection; or wherein the device (100, 200, 300, 400) or its object receiving unit (20) is configured to heat the food item by means of convectional aided heating.

**10.** A heating appliance for cooking a food item, comprising the device (100, 200, 300, 400) for determining sugar content information according to any of claims 1-9.

**11.** The heating appliance according to claim 10, wherein the heating appliance is an air-based fryer or an oven.

**12.** A method for determining sugar content information for a food item during or after cooking, comprising:

receiving the food item;
receiving a food type of the food item;
measuring color change of the food item during or after cooking; and
determining sugar content information for the food item on the basis of the food type and the measured color change.

**13.** The method according to claim 12, further comprising measuring weight of the food item as a function of time.

**14.** The method according to claim 13, further comprising determining an endpoint of cooking at least based on the weight loss rate of the food item which is dependent on weight change of the food item as a function of time.

**15.** A computer program element for controlling the device (100, 200, 300, 400) according to any one of claims 1 to 9 or the heating appliance according to any one of claims 10 to 11, which when executed by a processor is configured to carry out the method of any of claims 12-14.

FIG. 1

FIG. 2

FIG. 3

FIG. 4

FIG. 5

FIG. 6a

FIG. 6b

Europäisches
Patentamt

European
Patent Office

Office européen
des brevets

**EUROPEAN SEARCH REPORT**

Application Number

EP 17 17 8041

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2015/069325 A1 (MINVIELLE EUGENIO [US]) 14 May 2015 (2015-05-14) * figures 7, 8, 15, 16 * * paragraphs [0157] - [0168] * * paragraphs [0176], [0232], [0242], [0245], [0286] * | 1-15 | INV. G01N21/25 G01J3/46 G01J3/50 G01N33/02 |
| Y | US 2 498 024 A (BAXTER JOHN L) 21 February 1950 (1950-02-21) * column 2, line 24 - line 41 * | 1-15 | |
| Y | ROMANI S ET AL: "Image characterization of potato chip appearance during frying", JOURNAL OF FOOD ENGINEERING, BARKING, ESSEX, GB, vol. 93, no. 4, 20 February 2009 (2009-02-20), pages 487-494, XP026049403, ISSN: 0260-8774, DOI: 10.1016/J.JFOODENG.2009.02.017 [retrieved on 2009-02-20] * page 489, left-hand column, paragraph 4 - page 490, right-hand column, paragraph 1 * | 5 | |
| Y | NL 1 016 217 C2 (LEVENS GROUP B V [NL]) 21 March 2002 (2002-03-21) * page 7, line 36 - page 8, line 2; figures 1, 2 * | 8 | TECHNICAL FIELDS SEARCHED (IPC) G01N G01J F24C H05B |

The present search report has been drawn up for all claims

| Place of search | Date of completion of the search | Examiner |
|---|---|---|
| Munich | 14 November 2017 | Flentje, Farida |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 17 8041

This annex lists the patent family members relating to the patent documents cited in the above-mentioned European search report.
The members are as contained in the European Patent Office EDP file on
The European Patent Office is in no way liable for these particulars which are merely given for the purpose of information.

14-11-2017

| Patent document cited in search report | | Publication date | Patent family member(s) | | Publication date |
|---|---|---|---|---|---|
| WO 2015069325 | A1 | 14-05-2015 | EP JP KR WO | 3065561 A1 2017505106 A 20160082701 A 2015069325 A1 | 14-09-2016 16-02-2017 08-07-2016 14-05-2015 |
| US 2498024 | A | 21-02-1950 | NONE | | |
| NL 1016217 | C2 | 21-03-2002 | NONE | | |

EPO FORM P0459

For more details about this annex : see Official Journal of the European Patent Office, No. 12/82